# EUROPEAN PATENT APPLICATION

(11) **EP 2 687 603 A1**
(43) Date of publication of application: **22.01.2014**
(21) Application number: 12177111.7
(22) Date of filing: 19.07.2012
(51) Int. Cl.: C12N 15/67

(54) **T7 promoter variants and methods of using the same**

(71) Applicant: Ruhr-Universität Bochum, 44801 Bochum (DE)
(72) Inventor: Überla, Klaus, 45549 Sprockhövel (DE); Stang, Alexander, 42551 Velbert (DE); Siddartha, Paul, 44801 Bochum (DE)
(74) Representative: Viering, Jentschura & Partner

(57) **Abstract**

The present invention relates to T7 promoter variants with enhanced activity compared to the wild type as well as methods of using them. The present invention also relates to vectors and cells comprising said T7 promoter variants.

SEQUENCE LISTING

<110>  Ruhr-Universität Bochum

<120>  T7 promoter variants and methods of using the same

<130>  P50071

<160>  5

<170>  PatentIn version 3.5

<210>  1
<211>  10
<212>  DNA
<213>  Artificial Sequence

<220>
<223>  mutated T7 promoter sequence

<400>  1
aatcgssgag                                                              10


<210>  2
<211>  23
<212>  DNA
<213>  Artificial Sequence

<220>
<223>  mutated T7 promoter sequence

<400>  2
taatacgact cacaatcgss gag                                               23


<210>  3
<211>  10
<212>  DNA
<213>  Artificial Sequence

<220>
<223>  mutated T7 promoter sequence

<400>  3
aatcgcggag                                                              10


<210>  4
<211>  23
<212>  DNA
<213>  Artificial Sequence

<220>
<223>  mutated T7 promoter sequence

<400>  4
taatacgact cacaatcgcg gag                                               23


<210>  5
<211>  23
<212>  DNA
<213>  Bacteriophage T7

<400>  5
taatacgact cactataggg aga                                              23

## Description

### FIELD OF THE INVENTION

The present invention lies in the field of molecular biology and relates to T7 promoter variants with enhanced activity compared to the wild type as well as methods of using them. The present invention also relates to vectors and cells comprising said T7 promoter variants.

### BACKGROUND OF THE INVENTION

A huge number and variety of expression systems is known in the art which express the desired molecules in different hosts like mammalian, yeast, insect or bacterial cells, or are based on cell-free *in vitro* systems {Dyson and Durocher (2007) Expression Systems: Methods Express, 1st Ed., Cold Spring Harbor Laboratory Press, Cold Spring Harbor, NY}. Of particular interest are those expression systems which comprise T7 RNA polymerase and the corresponding T7 promoter. The T7 RNA polymerase and the T7 promoter from T7-like phages bear the ability for a very specific and efficient production of desired RNA molecules, both *in vitro* and in different host cells.

The T7 RNA polymerase is evolutionary and structurally related to the T3 and SP6 RNA polymerase but also to the mitochondrial RNA polymerase. Said polymerases catalyze the formation of RNA in the 5'→3' direction. However, in contrast to the multi-subunit family of RNA polymerases (including bacterial and eukaryotic sub-families) which also catalyze the formation of RNA in the 5'→3' direction, the T7 RNA polymerase family is characterized by being monomeric. Further characteristics of T7 RNA polymerases comprise the use of Mg²⁺ as an essential cofactor and sustained activity during treatment with the antibiotic rifampicin.

Transcription of the bacteriophage T7 genome which is unidirectional across the T7 chromosome requires two different RNA polymerases, *Escherichia coli* RNA polymerase and the T7 RNA polymerase. Early genes or class I genes are transcribed by *E*. *coli* RNA polymerase, while middle and late genes or class II and III genes are transcribed by T7 RNA polymerase {Ikeada, R.A. (1992) J. Biol. Chem. 267, 11322-11328}. Class II and III T7 promoters consist of highly conserved sequences of 23 continuous bases and seem to differ in their ability to stabilize the initiated polymerase-promoter complex, thus generating the strong T7 class III promoters and the weaker T7 class II promoters. The consensus sequence of the class III promoters is 5'-TAATACGACTCACTATAGGGAGA-3', wherein the transcription starts at position +1 (position +1 being the first G in GGGAGA). These promoter regions present in the DNA of bacteriophage T7 provide the interesting effect that bacterial cells infected by this bacteriophage almost instantly produce only T7 bacteriophage polypeptides, to the virtual exclusion of the host cell's own requirements. This action is in large measure a consequence of the very strong interaction between the T7 promoter and the T7 RNA polymerase.

In academic science and industrial biotechnology expression via T7 RNA polymerase is a highly popular tool based on the low error rate of the T7 RNA polymerase, the extremely promoter-specific expression and the low basal expression activity. Further, the T7 RNA polymerase offers the possibility to only transcribe DNA downstream of the T7 promoter.

Based on the low error rate and the high efficiency, the T7 RNA polymerase and T7 promoter expression system also provides, in addition to its use in host cells, a platform for *in vitro* transcription of RNA or coupled *in vitro* transcription/translation reactions. *In vitro* expression systems are often used for synthesis of RNA or when only small amounts of the desired molecule are required. Moreover, these systems allow for a rapid identification of genes and their products and offer advantages in the expression of toxic products. Nowadays, several different efficient *in vitro* expression systems are known in the art, with the rabbit reticulocyte lysate, the wheat germ extract and the *E. coli* cell-free system representing the most popular and well-characterized systems.

Over recent years, different methods to further reduce the basal expression activity of T7 RNA polymerase, like coexpression with lysozyme which inhibits T7 RNA polymerase activity or the control of the T7 RNA polymerase gene under tight promoters, have been established. Wycuff and Matthews {Wycuff and Matthews (2007) Anal. Biochem., 277, 67} disclose an expression method in which the T7 RNA polymerase gene is under the control of the inducible arabinose promoter leading to a lower basal expression. However, the induction of T7 RNA polymerase expression by arabinose is connected with higher costs compared to the commonly used induction by isopropyl-β-D-thiogalactopyranosid (IPTG). U.S. Patent Application No. 5,547,862 describes the use of a 5' β-globin leader sequence and synthetic poly(A)₃₀ tail downstream of the T7 promoter for enhanced *in vitro* transcription. However, the expression vectors disclosed in this document still rely on the wild type sequence of the T7 promoter.

Hence, there is need in the art for materials and methods that allow improving the activity of the T7 promoter, in particular for its use in *in vitro* based expression systems.

### SUMMARY OF THE INVENTION

The present invention is based on the inventors' finding that variants of the T7 promoter that comprise the nucleotide sequence set forth in SEQ ID NO:1 or 2 exhibit increased transcriptional efficiency.

In a first aspect, the present invention is thus directed to an isolated nucleic acid molecule comprising or consisting of one or more nucleotide sequences selected from the group consisting of SEQ ID NO:1 and SEQ ID NO:2 and complements thereof. In SEQ ID NO:1 or 2, each S at positions 6 and 7 of SEQ ID NO:1 and each S at positions 19 and 20 of SEQ ID NO:2 is independently selected from C and G.

In specific embodiments of this first aspect of the invention, S at position 6 of SEQ ID NO:1 and S at position 19 of SEQ ID NO:2 are C and S at position 7 of SEQ ID NO:1 and S at position 20 of SEQ ID NO:2 are G.

In further embodiments of the invention, the nucleotide sequence is SEQ ID NO:2.

In some embodiments of the invention, the isolated nucleic acid molecule comprises, in addition to the sequence of SEQ ID NO:1 or 2 or complements thereof, at least one of a nucleotide sequence encoding for an open reading frame of a gene of interest, a -35/- 10 box, a Shine-Dalgamo element, a TATA box, a CAAT sequence, a 5'-capping element, enhancer and/or repressor element(s), signal and/or leader sequence(s), a protein binding sequence, and a restriction enzyme site. Also encompassed are embodiments where the isolated nucleic acid molecule comprises more than one of these additional sequences. If the additional sequence is a gene of interest, it is preferably downstream of the sequence of SEQ ID NO:1 or 2 or complements thereof.

In a second aspect, the invention relates to a vector comprising the isolated nucleic acid molecule according to the invention.

In some embodiments of the invention, the vector is a mammalian expression vector, a bacterial plasmid, a yeast episomal vector, an artificial chromosomal vector, or a viral vector.

In a third aspect, the present invention also relates to a host cell comprising the isolated nucleic acid molecule or the vector according to the invention.

In specific embodiments of the invention, the host cell is a eukaryotic cell.

In further embodiments of the invention, the host cell is a prokaryotic cell.

The invention is also directed to a method of producing RNA or protein comprising contacting the isolated nucleic acid molecule or the vector according to the invention with an *in vitro* transcription translational reaction (IVTT) or an *in vitro* transcription reaction , or introducing the isolated nucleic acid molecule or vector according to the invention into a host cell, for example as defined above, under conditions that allow transcription and optionally translation of the isolated nucleic acid molecule.

In specific embodiments of the invention, the method comprises contacting the isolated nucleic acid molecule or the vector according to the invention with an *in vitro* transcription translational reaction (IVTT) or an *in vitro* transcription reaction.

In various embodiments of the invention, the *in vitro* transcription translation reaction is a rabbit reticulocyte lysate.

In a further aspect, the invention is directed to a kit comprising the isolated nucleic acid molecule or the vector according to the invention.

### BRIEF DESCRIPTION OF THE DRAWINGS

The invention will be better understood with reference to the detailed description when considered in conjunction with the non-limiting examples and the accompanying drawings.

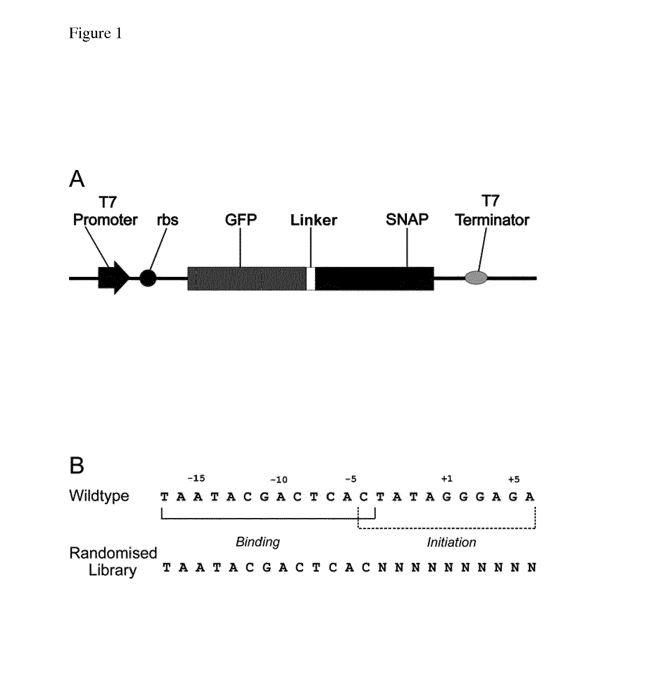

Figure 1 shows templates for multicopy bead display approach. (A) Map of the expression cassette. The open reading frame for GFP is fused in frame via a flexible linker region to the open reading frame of SNAP. The cassette is flanked by the T7 promoter, a ribosome binding side (rbs) and a T7 terminator sequence. (B) Sequence of the wild type and the randomized T7 promoter. The polymerase binding site and the transcription initiation site are indicated.

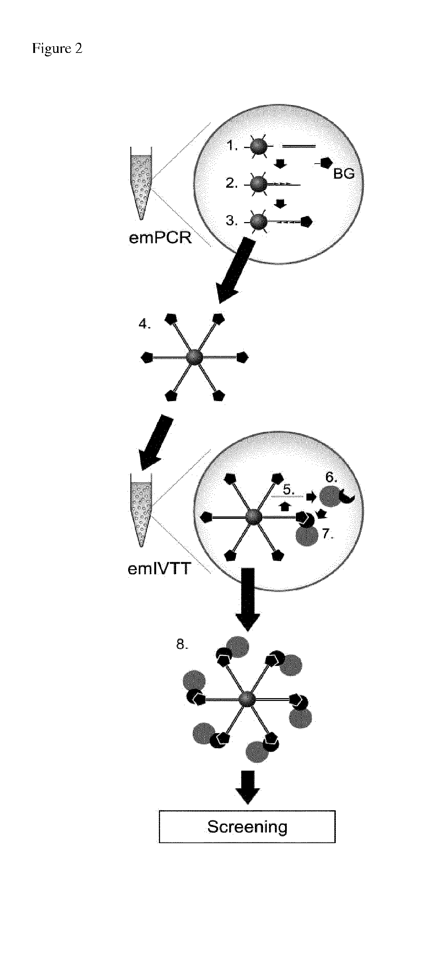

Figure 2 schematically shows the principal steps of the multicopy bead display approach. The bead display approach involves two steps of compartmentalization using water in oil emulsions. In the first step, single template molecules of a DNA library are amplified in each picoliter reactor of an emulsion PCR (emPCR) with oligonucleotides coupled to microbeads and other oligonucleotides covalently coupled with benzylguanine (BG) moieties (steps 1-3). As a result many copies of the same DNA each carrying a BG substrate are immobilized on the bead. These beads are then recovered from the water in oil emulsion (step 4) and used in a second step in an *in vitro* transcription translation reaction also performed in emulsion. The amplicons contain the T7 phage promoter upstream of an open reading frame encoding a fusion protein under selection and the modified DNA repair protein 06-alkylguanine-DNA alkyltransferase (SNAP) that reacts specifically with BG moieties incorporated into the amplicons. During the transcription translation reaction the fusion proteins synthesized in each picoliter reactor of the emulsion (step 5 and 6) are covalently coupled to the BG moieties of the beads present in the same picoliter reactor. The beads can then be isolated from the emulsion (step 8) and used in subsequent screening assays.

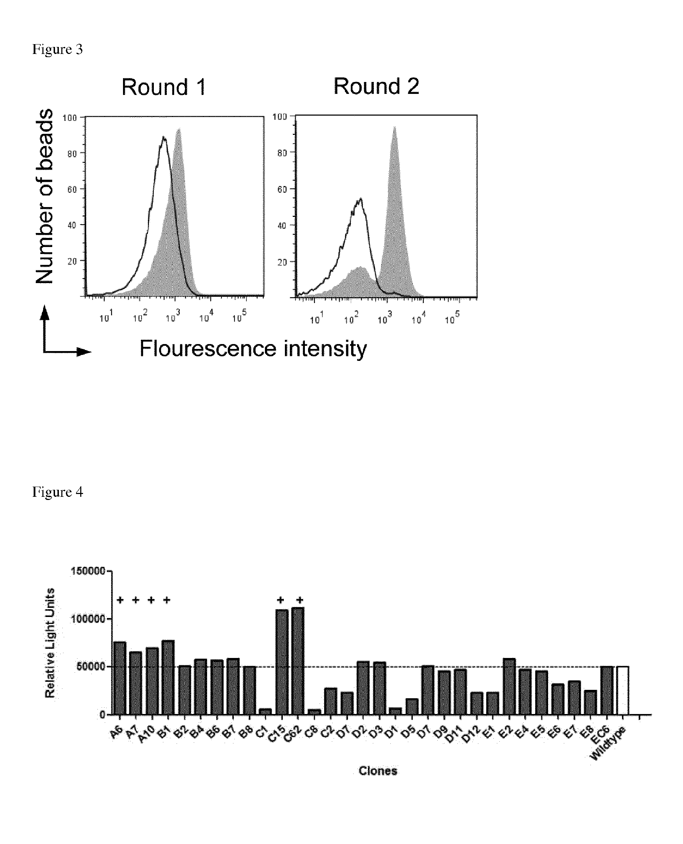

Figure 3 shows results of the selection process to isolate variants with the highest activity from the T7 promoter library. Beads were loaded with amplicons from the randomized T7 promoter library upstream of the GFP-SNAP coding region by emulsion PCR, transferred to compartmentalized IVTT reactions, stained for GFP and analysed by FACS. Overlays of histograms of flow-cytometric analyses during yield (black line) and purity sort (grey area) of the first and second round of selection are shown.

Figure 4 shows results of the activity of promoter variants selected from the T7 promoter library by multicopy bead display. The T7 promoter variants from the purity sort of the second round of selection were loaded on beads by a third round of emulsion PCR. Beads were transferred to a compartmentalized IVTT reaction and then stained for GFP. Single beads were sorted by flow-cytometry into wells of a 96 well plate and the T7 promoter region of single beads was amplified by PCR and used to express the luciferase by IVTT. The reporter gene activity of 31 T7 promoter variants obtained from selected single beads is shown in comparison with the wild type T7 promoter.

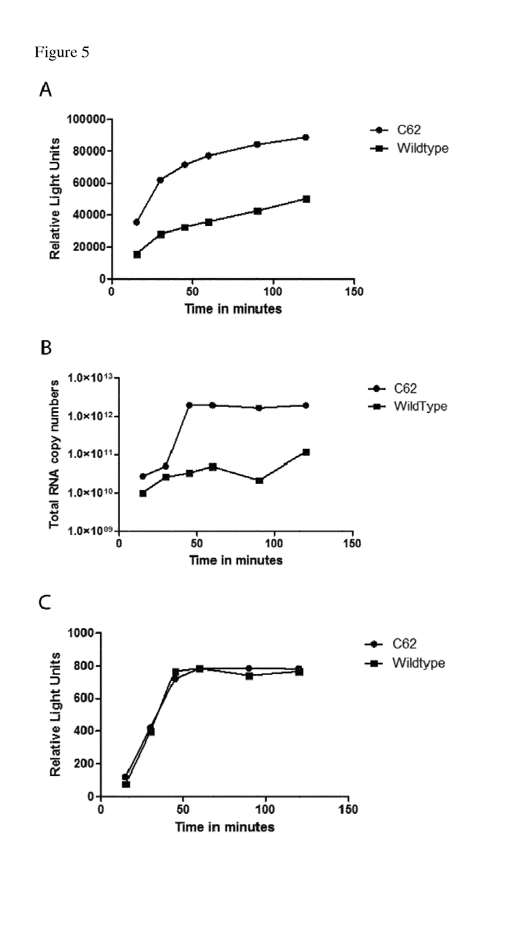

Figure 5 shows the transcriptional and translational activity of mutant and wild type T7 promoters. (A) Time course of the luciferase activity in IVTT reactions of the luciferase gene driven by the C62 or wild type T7 promoter. (B) Time course of luciferase RNA levels in *in vitro* transcription reactions of the luciferase gene driven by the C62 or wild type promoter. (C) Time course of luciferase activity of *in vitro* translation reactions of equal amounts of luciferase mRNA containing either the first transcribed nucleotides of the wild type or the C62 T7 promoter. Results of one representative experiment out of 2 independent experiments are shown.

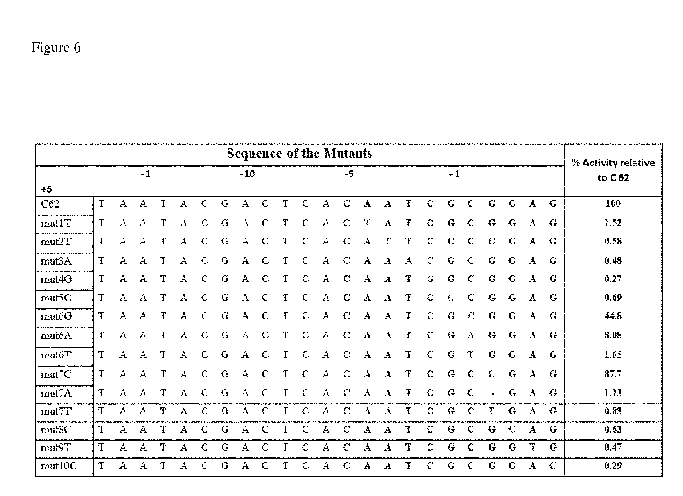

Figure 6 shows the characterization of the C62 T7 promoter derivatives. The C62 T7 promoter was mutated by separately replacing each nucleotide of the randomized sequence. Usually, A was mutated to T, T was mutated to A, G was mutated to C and C was mutated to G. For each of the C62 T7 promoter based derivatives the luciferase RNA levels after 40 min of transcription and translation by IVTT reactions were measured. The results are shown as relative activities adjusted to the activity of the C62 T7 promoter.

### DETAILED DESCRIPTION OF THE INVENTION

The terms used herein have, unless explicitly stated otherwise, the following meanings.

The term "T7 promoter", as used herein, relates to the promoter sequence of late transcribed (phase III) bacteriophage T7 genes. The nucleic acid sequence of the T7 promoter is 23 nucleotides long and set forth in SEQ ID NO:5. This sequence is herein also called "wild type sequence" or "wild type". Said sequence is recognized by the T7 RNA polymerase, wherein the polymerase can initiate transcription in a variety of different host cells, like bacterial cells, mammalian cells, plant cells or insect cells when the T7 RNA polymerase and the T7 promoter bind to each other in said cell.

The term "increased (transcriptional or translational) efficiency/activity" as used herein in relation to the mutated T7 promoter sequences, means that promoter increases transcription and/or translation, as for example detectable via the RNA amount or protein amount produced over a defined period of time, compared to the respective wild type sequence, i.e. usually the sequence set forth in SEQ ID NO:5.

The term "isolated nucleic acid molecule", as used herein, relates to DNA (deoxyribonucleic acid) or RNA (ribonucleic acid) molecules. Said molecules may appear independent of their natural genetic context and/or background and are preferably separated from other nucleic acids or cellular components. The separation may occur by purification, for which various techniques are known in the art. The term "nucleic acid molecule" further refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms.

"At least one", as used herein, relates to one or more, in particular 1, 2, 3, 4, 5, 6, 7, 8, 9, 10 or more.

The term "sequence", as used herein, relates to the primary sequence of nucleic acid molecules.

"Complement", as used herein, relates to a nucleic acid molecule which is complementary to another nucleic acid molecule when both nucleic acid molecules are aligned antiparallel to each other in that one or more or all nucleotides of either of the nucleic acid molecules forms Watson-Crick base pairs with their corresponding counterparts. In various embodiments, the complements are full complements in that each nucleotide of the respective molecule or sequence forms a Watson-Crick base pair with a corresponding nucleotide on the other strand.

The term "S" as used herein represents the nucleotides guanosine or cytidine. The base of guanosine, guanine, belongs to the group of purines whereas the base of cytidine, cytosine, is a pyrimidine derivate. Both bases form hydrogen bonds to each other in Watson-Crick base paring and this binding is viewed as the stronger Watson-Crick base pair over the pairing of adenine and thymine. Generally, reference is made herein to the nucleotides by referring to their established one letter code, i.e. A means adenosine, T means thymine, C means cytidine, G means guanosine, and U means uridine.

"Ligated", as used herein, means the end-to-end joining of two or more nucleic acid segments, wherein the 5'phosphate end of a (poly)nucleotide is coupled to the 3'hydroxyl end of another (poly)nucleotide with the help of a DNA ligase to form a phosphodiester bond.

The term "3'end" or "3'hydroxyl end" as interchangeably used herein relates to the termination at the hydroxyl group of the third carbon in the sugar-ring of a nucleic acid molecule, and is also known as the tail end. The term "5'end" or "5'phosphate end" as interchangeably used herein designates the end of the DNA or RNA strand that has a phosphate group at the fifth carbon in the sugar-ring of the deoxyribose or ribose at its terminus.

In embodiments where the isolated nucleic acid molecule comprises an additional nucleotide sequence, said sequence may be of bacterial, mammalian, plant, insect and nematode origin. Said nucleic acid sequence may comprise or consist of, but is not limited to one or more sequences encoding for an open reading frame of a gene of interest, a - 35/-10 box, a Shine-Dalgamo element, a TATA box, a CAAT sequence, a 5'-capping element, enhancer and/or repressor elements, signal and/or leader sequences, protein binding sequences and restriction enzyme sites.

"Vector" as used herein relates to genetically manipulated, cyclic, usually double-stranded nucleic acid molecules which allow specific selection and the directed introduction of genetic material of interest. Preferably, such vectors are mammalian expression vectors, bacterial plasmids, bacteriophage vectors, yeast episomal vectors, artificial chromosomal vectors or viral vectors.

The term "host cell" or "host" as interchangeably used herein means an organism that harbors the isolated nucleic acid molecule or vector according to the present invention. Such host cell can be a mammalian cell, a plant, a bacterium, an insect cell or a nematode. Preferably, the host is a eukaryotic cell. In another embodiment said host is a prokaryotic cell.

"RNA" or "ribonucleic acid" as interchangeably used herein relates to a chain of nucleotides wherein the nucleotides contain the sugar ribose and bases selected from the group of adenine (A), cytosine (C), guanine (G), or uracil (U). "DNA" or "deoxyribonucleic acid" as interchangeably used herein relates to a chain of nucleotides wherein the nucleotides contain the sugar 2'-deoxyribose and bases selected from adenine (A), guanine (G), cytosine (C) and thymine (T).

"Protein" as used herein is defined as consisting of one or more polypeptides, wherein the polypeptides consist of amino acids coupled by peptide (amide) bonds. The term polypeptide refers to a polymeric compound comprised of covalently linked amino acid residues. The amino acids are preferably the 20 naturally occurring amino acids glycine, alanine, valine, leucine, isoleucine, phenylalanine, cysteine, methionine, proline, serine, threonine, glutamine, asparagine, aspartic acid, glutamic acid, histidine, lysine, arginine, tyrosine and tryptophan.

The term "contacting" as used herein means that two or more components are brought together to allow direct interaction. Such interaction or contact may take place under diverse conditions, such as liquid media, gels or air. Said conditions are non-limiting.

The term "introducing the isolated nucleic acid molecule or vector" as used herein relates to methods that allow the transfer of nucleic acid over the cell membrane and/or cell wall into the cytoplasm and, depending on the cell type, the nucleus. Methods that allow the transfer of nucleic acid molecules into cells are known in the art and comprise, for example, competent cells, electroporation, gold particle gun, liposome-based transfection reagents and viral infections.

"IVTT reaction" or "in vitro transcription translation reaction" as interchangeably used herein relates to cell-free systems that allow for specific transcription and translation by comprising macromolecular components (RNA polymerase, 70S or 80S ribosomes, tRNAs, aminoacyl-tRNA synthetases, initiation, elongation and termination factors, etc.) required for transcription and translation. To ensure efficient translation, the system may also be supplemented with amino acids, energy sources (ATP, GTP), energy regenerating systems, and other co-factors (Mg²⁺, K⁺, etc.). Such systems or extracts are also known as "coupled" and "linked" systems as they start with DNA templates, which are subsequently transcribed into RNA and then translated. Preferred IVTT reactions comprise the rabbit reticulocyte lysate, the wheat germ extract and the *E*. *coli* cell-free system, in a more preferred embodiment the IVTT reaction is the rabbit reticulocyte lysate. Further, the term "in vitro transcription reaction" as used herein is directed to a DNA template including the T7 promoter variants according to the present invention, the supplements and co-factors as described above (including nucleoside triphosphates) and purified, preferably recombinantly expressed, T7 RNA polymerase.

"Kit", as used herein, relates to a kit-of-parts wherein the separate components of the kit are physically separated as individual components.

The inventors of the present invention have unexpectedly found that T7 promoters consisting of the nucleic acid sequence as set forth in SEQ ID NO:2 and SEQ ID NO:4 have increased activity compared to the wild type consensus sequence SEQ ID NO:5. The T7 promoter variants according to the present invention comprise, compared to the wild type consensus sequence of the T7 promoter, as set forth in SEQ ID NO:5, 6 different nucleotides in the region of the last 10 nucleotides of the 3' hydroxyl end. The increased activity can be observed in IVTT reactions. In a preferred embodiment of the invention, the IVTT reaction is rabbit reticulocyte lysate. Moreover, the inventors identified two positions in the sequence of the T7 promoter variant that allow the replacement by guanine or cytosine and still maintain increased activity.

As the last 10 nucleotides of the 3'hydroxyl end of the T3 promoter are identical to 9 nucleotides of the 3' hydroxyl end of the consensus T7 promoter and said promoters only differ at one position, namely position -2 (T7: thymidine; T3: adenine), it is assumed that the T7 promoter variant according to the present invention also increases the promoter activity in the context of a T3 promoter sequence and/or T3 RNA polymerase.

It is further found by RNA isolation experiments that the increased T7 promoter activity of the present invention is caused by 10-times increased transcriptional activity. In terms of translational activity, the T7 promoter variant according to SEQ ID NO:4 shows identical expression levels compared with the T7 promoter wild type in a reporter gene expression system.

In order to detect the enhanced T7 promoter activity of the present invention a reporter gene can be used. Preferred reporter genes include, but are not limited to luciferase, green fluorescent protein (GFP), peroxidase, chloramphenicolacetyltransferase (CAT) and the *E. coli* gene lacZ. In a more preferred embodiment, the reporter gene is luciferase.

In specific embodiments of the invention, the isolated nucleic acid molecule according to the invention additionally comprises, for example, a nucleic acid sequence encoding for a gene of interest and/or sequences that allow its insertion into a vector, and can be cloned in a known host organism. Several cloning techniques, including amplification of nucleic acids, their restriction by according enzymes, purification and ligation, and transformation techniques, are known in the art and described in more detail by Sambrook et al.{Sambrook, J. et al. (1989) Molecular Cloning: A Laboratory Manual, 2nd Ed., Cold Spring Harbor Laboratory Press, Cold Spring Harbor, NY}. The produced nucleic acid constructs are verified by sequencing. Sequencing of the nucleic acid constructs can be done by the chain termination method, Sanger sequencing or Maxam-Gilbert sequencing or any other technique known in the art. Alternatively, high-throughput sequencing, like pyrosequencing, SOLiD sequencing or DNA nanoball sequencing, is used to determine the sequence of the nucleic acid molecules of the present invention {Alphey, L. (1997) DNA Sequencing: From Experimental Methods to Bioinformatics, 1st Ed., Bios Scientific Pub Ltd., Oxford, UK}.

In another aspect, the invention relates to a vector comprising the isolated nucleic acid molecule according to the invention. In specific embodiments, a host cell is transformed with the vector comprising the nucleic acid molecules according to the present invention. Methods for the transformation of host cells include, but are not limited to competent cell based techniques, electroporation, gold particle gun based techniques, liposome-based transfection reagents and bacterial or viral infections. The host cells comprise bacterial cells, yeast cells, plant cells, nematode cells, insect cells and mammalian cells. The transfection method of choice can vary dependent on the host cell {The QIAGEN Transfection Resource Book (2002), 2nd Ed., QIAGEN GmbH, Hilden, Germany}.

In a further aspect of the present invention, nucleic acids according to the present invention are used in methods to produce RNA. In these methods the RNA can be synthesized in cell-free *in vitro* systems like the rabbit reticulocyte lysate, the wheat germ extract and the *E. coli* cell-free system or in an *in vitro* transcription reaction or in a host cell. Protocols including conditions for RNA synthesis and subsequent purification and storage conditions are described in the prior art, for example by Krieg {Krieg, P. (1996) A Laboratory Guide to RNA: Isolation, Analysis, and Synthesis, 1st Ed., Wiley-Liss, Hoboken, New Jersey}.

In specific embodiments, the present invention relates to methods of producing protein using cell-free *in vitro* systems or host cells and the nucleic acid molecules and vectors according to the present invention. Host cells for the synthesis of protein comprise bacterial, yeast, plant, nematode hosts as well as insect or mammalian cells. In specific embodiments, the host is *E. coli, Pichia pastoris,* a HeLa cell or a SF9 cell. Detailed protocols including system specific synthesis conditions and purification methods can be found for recombinant cell-based expression in Merten et al. {Merten et al. (2001) Recombinant Protein Production with Prokaryotic and Eukaryotic Cells: A Comparitive View on Host Physiology, 1st Ed., Kluwer Academic Publishers, Dordrecht, The Netherlands) and for cell-free expression in Spirin and Swartz (Spirin, A.S. and Swartz, J.R. (2008) Cell-Free Protein Synthesis: Methods and Protocols, 1st Ed., Wiley-VCH Verlag GmbH, Weinheim, Germany).

In order to select single cells or specific population of cells, for example cells expressing specific levels of a reporter gene, flow cytometry can be used. In specific embodiments, fluorescence-activated cell sorting (FACS) is able to measure the reporter gene GFP by detecting antibodies conjugated to GFP or farnesylated GFP and subsequently sorting the cells expressing said reporter gene by different parameters, as for example, the expression level of the reporter gene. Other reporter genes to be measured are luciferase, peroxidase and the chloramphenicolacetyltransferase (CAT). Methods to detect and quantify the expression of these reporter genes comprise substrate specific fluorescence or luminescence assays.

To efficiently test the T7 promoter library generated by the present inventors, nucleic acid molecules are coupled to microbeads or benzylguanine. Conditions and steps to ensure efficient coupling are known in the art {Kojima, T. et al. (2009) Nucleic acids research 33, e150}.

In order to identify expression levels of genes of interest, real-time PCR (RT-PCR) is used. This method allows both, detection and quantification, of nucleic acid molecules. It is known in the art that real-time PCR measurements can be combined with reverse transcription to quantify messenger RNA and non-coding RNA. Protocols for measurements via RT-PCR are described by VanGuilder et al. {VanGuilder, H.D. et al. (2008) Biotechniques 44, 619-626}

The present invention is further illustrated by the following examples. However, it should be understood, that the invention is not limited to the exemplified embodiments.

### EXAMPLES

### Example 1: T7 promoter library construction

A T7 promoter library was constructed by PCR amplification of a cloned open reading frame encoding a GFP-SNAP fusion protein with oligonucleotides T7-wobble-s 5'-AGCAACCGCACCTGTGGTAATACGACTCACNNNNTTGTGAGCGGATAA CAATTCCCC-3' (wherein N is G, C, T or A) and T7Term-a 5'-GGATATAGTTCCTCCTTTCAGC-3'. The oligonucleotide T7-wobble-s cover the T7 promoter and adjacent sequences in which 10 nucleotides flanking the transcription initiation site of the T7 promoter are completely randomized (Figure 1B). As the PCR template further comprises a spacer and oligonucleotide binding site downstream of the ORF of the GFP-SNAP fusion gen, the PCR product is a library of randomized T7 promoter variants followed by the GFP and SNAP coding regions. The wild type T7 promoter construct was produced using T7-consensus-s oligonucleotide 5'-AGCAACCGCACCTGTGGTAATACGACTCACTATAGGGAGATTGTGAGCGGATAA CAATTCCCC-3' (without any randomization in the T7 promoter) and oligonucleotide T7Term-a, thereby maintaining the same reading frame as the library construct. PCR products were run on an agarose gel and eluted to completely remove the template plasmid DNA. The randomization of the 10 nucleotides was confirmed by sequencing the PCR product. The resulting constructs comprise the randomized T7 promoter upstream of the open reading frame of the GFP and SNAP coding regions (Figure 1A).

### Example 2: Multicopy bead display approach

The multicopy bead display approach (Fig. 2) involves two steps of compartmentalization using water in oil emulsions and is basically covered by steps of covalent coupling of oligonucleotides to microbeads and benzylguanine, water in oil emulsion PCR and cell-free expression of proteins in water in oil emulsion.

*Covalent coupling of oligonucleotides to microbeads and benzylguanine:* 5'-amino-modified oligonucleotides 5'-TGTGTGTGTGTGTGTGTGTGTGTGTGTGTGAGCAACCGCACCTGTGG-3' or 5'-AGCAACCGCACCTGTGG-3' were coupled to magnetic beads (Dynabeads Myone carboxylic acid, Invitrogen) according to the manufacturer's guidelines. In brief, 7-12×10⁸ beads (100 µl of the bead solution) were transferred into 1.5 ml eppendorf tubes and washed three times with 100 µl of 0.01 N NaOH and three times with 100 µl deionized water. Beads were then resuspended in 50 µl 25 mM MES (2-[N-morpholino] ethane sulfonic acid, pH 6.0) buffer. 20 µl of a 100 µM 5'-amino modified oligonucleotide solution was added to this solution and incubated on an end-over-end rotator for 30 min. After 30 min 3 mg of EDC (3-dimethylaminopropyl carbodiimide hydrochloride) dissolved in 30 µl of 25 mM MES buffer (pH 6.0) were added and incubated overnight on an end-over-end rotator at 4° C. Beads were then washed 5 times with 100 µl TE (pH 8.0) and stored in 100 µl of TE buffer at 4° C. The oligonucleotide 5'-GGATATAGTTCCTCCTTTCAGC-3' was chemically coupled to benzylguanine substrate (New England Biolabs) using a protocol as described by Stein et al. {Stein et al. (2007) Chembiochem, 8, 2191-2194}.

*Water in oil emulsion PCR:* The aqueous phase is composed of 100 µl PCR mix comprising the following reagents: 3×10⁹ copies of linear template DNA, 7-12×10⁷ beads coupled with oligonucleotides, 0.01 µM of oligonucleotide 5'-AGCAACCGCACCTGTGG-3', 3 µM of benzylguanine coupled oligonucleotides, 0.5 mM of dNTPs, 15 U of DreamTaq DNA polymerase (Fermentas) and 10 µl of BSA (10 mg/ml). The mineral oil mixture containing 2 % Abil-90 EM and 0.05 % Triton X-100 was prepared as described in the prior art {Williams, R. et al. (2006) Nature Methods, 3, 545-550}. In a 2 ml cryovial with flat bottom the aqueous phase was gradually added drop wise to 500 µl of the oil phase over a period of 5 min while stirring constantly on ice at 2000 rpm. Stirring was continued for another 3 min to obtain a homogenous emulsion. The emulsion was then dispensed in 50 µl aliquots into ten 200 µl thin-walled PCR tubes. PCR was then performed in a conventional thermocycler with the following temperature profile: 95° C for 3 min, 45 cycles of 95° C for 30 s, 58° C for 30 s, 72° C for 90 s, respectively followed by a final extension at 72° C for 10 min. After PCR, all samples were collected in a 1.5 ml eppendorf tube and centrifuged at 13,000 g for 5 min. The oil was removed from the top and the beads with yet intact emulsion remained at the bottom. The emulsion was disrupted by adding 100 µl of Bind and Wash buffer [10.0 mM Tris-HCl (pH 7.5), 1.0 mM EDTA, 2.0 M NaCl], 1 ml of hexane and vortexing for 10 seconds. The disrupted oil phase (top) in hexane was removed and discarded and beads settled down in the aqueous layer. To completely remove the oil from the bead suspension, hexane extraction was repeated three more times. Residual hexane was removed by centrifugation under vacuum at room temperature for 5 min. The beads were then washed 5 times with TE pH 8.0 and resuspended in 9 µl of nuclease free water.

*Cell-free expression of proteins in water in oil emulsion:* The TNT T7 Quick for PCR DNA system (Promega) was used to perform *in vitro* transcription-translation reaction in emulsion. 50 µl of reaction mixes were assembled on ice by combining 40 µl of TNT T7 Quick for PCR DNA mix supplemented with 1 µl of methionine (1 mM) and 9 µl of beads suspended in nuclease free water. The reaction mixture was added to the oil mixture as described above for the emulsion PCR to form the emulsion IVTT. The emulsion was then incubated at 30° C for 30 min for the expression of proteins. The emulsion was put on ice for 5 min and breaking of emulsion was done as described by Miller et al. (Miller, O.J. (2006) Nature Methods, 3, 561-570}. 100 µl of breaking buffer (PBS containing ImM DTT and 10 pM benzylguanine-NH2) was also added during the recovery of beads. The use of DTT is for protein stability and benzylguanine-NH2 was added to remove the unbound proteins expressed in picoliter reactor to avoid binding on beads with unsaturated amounts of proteins on them. The beads were then washed twice with breaking buffer and resuspended in 100 µl of FACS buffer (PBS + 0.5% BSA).

### Example 3: Selection of T7 promoter variants with enhanced activity

To select beads with the highest GFP fluorescence and to avoid selection of false positive beads a two-step flow-cytometric sorting strategy was used. During a first yield sort, around 1% of the beads with the highest fluorescence intensities was selected and then submitted to a more accurate purity sort selecting again around 1% of the remaining beads with the highest fluorescence intensity. In detail, for yield and purity sort 100 µl of a 1:100 dilution of anti-GFP rabbit serum (Invitrogen) in FACS buffer were added to the beads coupled with the GFP-SNAP fusion protein mixed well and incubated at room temperature for 1 h with intermittent mixing every 20 min. The beads were then washed twice with 100 µl of FACS buffer and stained with 100 µl of 1:100 diluted anti rabbit IgG (Invitrogen) for 1 h in the dark at room temperature with intermittent mixing every 20 min. The beads were then washed two times with 100 µl of FACS buffer, resuspended in 500 µl FACS buffer and analyzed in a FACS Calibur (Bectin Dickenson). For the screening, beads displaying the library were stained with antibodies as described above and were sorted in a FACS Di VA cell sorter (Bectin Dickenson) after gating on single beads by forward and side scatter. Each round of sorting protocol involved two sorts. Beads were first yield sorted with a speed of 5000-10000 beads/s. After yield sort, sorted beads underwent purity sort which was performed at a speed below 2000 beads/s. The purity sort beads were directly collected in 50 µl nuclease free water for subsequent PCR amplification. Beads recovered from purity sort were used to amplify the randomized T7 promoter region by PCR. The amplified T7 promoter region was fused by overlap extension PCR to the remaining GFP-SNAP expression cassette regenerating the entire GFP-expression cassette shown in Figure 1A. This PCR product was excised from the gel and a second round of multicopy bead display was performed. Analyzing the fluorescence intensity distribution of the beads after the first and the second round of selection reveals an increase in mean fluorescence intensity from yield sort to purity sort (Figure 3). The T7 promoter region of the beads recovered from the purity sort during the second round was amplified and used to reconstitute the entire expression cassette as described above. Single GFP-positive beads obtained after a third round of the multicopy bead display were directly sorted into microliter PCR plates.

### Example 4: IVTT activity of T7 promoter variants and wild type

The activity of the selected T7 promoter regions was determined by fusing said promoter to the luciferase gene by overlap extension PCR. For construction of vectors comprising a luciferase gene under the control of a T7 promoter, the mutant T7 promoter sequences were PCR amplified using the oligonucleotides 5'-AGCAACCGCACCTGTGG-3' and 5'-ATGTTTTTGGCGTCTTCCATGGTATATCTCCTTCTAAAGTTAAACAAA-3' to confer each of these variants with a segment of the luciferase gene for subsequent overlap extension PCR. The PCR was performed using Faststart Taq polymerase (Roche) with following temperature profile: 94° C for 4 min, 25 cycles of 94° C for 15 s, 60° C for 30 s, 72° C for 30 s followed by final extension at 72° C for 7 min. The luciferase gene without any promoter was amplified from the template plasmid with the oligonucleotides 5'-ATGGAAGACGCCAAAAACAT-3' and 5'-CATTTAGGTGACACTATAGAATAGGG-3' using Faststart Taq polymerase (Roche) with following temperature profile: 94° C for 4 min, 25 cycles of 94° C for 15 s, 60° C for 30 s, 72° C for 90 s followed by final extension at 72° C for 7 min. Both of these fragments were then mixed in equimolar concentration to perform the overlap extension PCR to produce the entire expression cassettes for the luciferase gene under control of different T7 promoter variants. This PCR was performed using Dreamtaq polymerase (Fermentas) with the following temperature profile 94° C for 3 min, 35 cycles of 94° C for 30 s, 62° C for 30 s, 72° C for 90 s, respectively followed by final extension at 72° C for 10 min. Products were gel purified and the concentration was determined photometrically.

*IVTT activity of T7 promoter variants:* The luciferase gene under the control of different promoter variants was expressed using the TNT T7 Quick for PCR DNA kit (Promega). One fifth of one reaction was used for analysis of each template. 200 ng of DNA were mixed with 8 µl of master mix containing 1 mM of methionine in 0.5 ml tubes. Expression of proteins was done at 30° C for 15-120 min. After expression, the reactions were snap chilled in ice for 5 min prior to depleting the reaction of RNA by adding 0.5 µl of RNase IF (New England Biolabs) and incubating the reaction at 37° C for 30 min. Expression levels of luciferase were analyzed in a luminometer using Bright Glo luciferase substrate (Promega) as described by the manufacturer. About half of the amplifiable T7 promoter regions had an activity that was as high as or higher than the wild type T7 promoter included as a positive control (Fig. 4). The activity of 40 randomly-picked clones of the T7 promoters of the original library was also determined (data not shown). None of the clones had an activity close to the one of the wild type promoter. Around 20 % of the wild type promoter's activity was detected in 4 of the random clones. Thus, the high activity of the T7 promoters selected by the multicopy bead display is not a random effect but due to the selection procedure.

The six T7 promoters with the highest activity were then sequenced directly from amplicons. Since two sequences were represented by two independently selected beads, a total of four different T7 promoter sequences were obtained with a higher activity than the wild type promoter sequence (Table 1). The only nucleotide of the randomized region conserved between the wild type T7 promoter and all four selected sequences is the guanine at position +1.

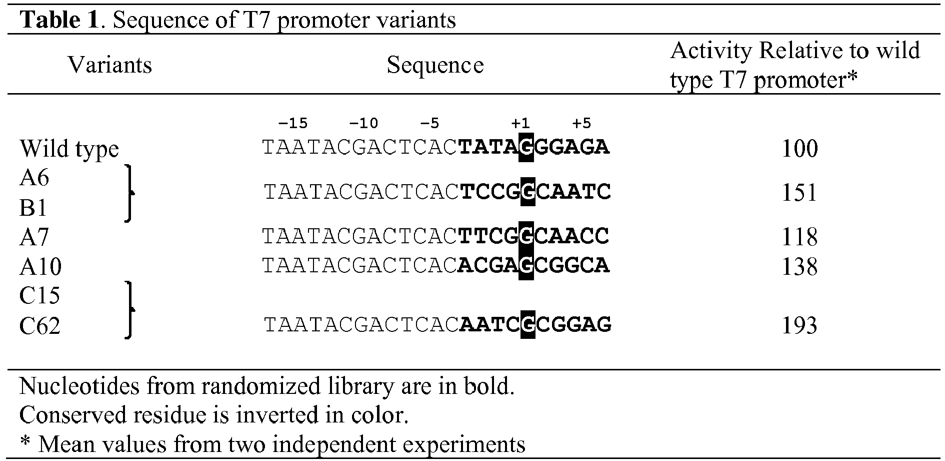

### Example 5: Activity of C62 T7 promoter variant

The T7 promoter variant with the highest activity (C62) was further characterized in direct comparison to the wild type T7 promoter. The luciferase activity of these variants was determined at different time points after starting the IVTT. The C62 T7 promoter led to a least 2-fold higher protein expression levels at each of the time points tested (Fig. 5A). Luciferase expression levels obtained by wild type T7 promoter after 1 hour were already obtained with the C62 T7 promoter after 15 min.

*Transcriptional activity of C62 T7 promoter variant:* To assess the relative transcriptional activity, RNA was isolated from IVTT reactions with the wild type and the C62 T7 promoter at different time points and the amount of RNA was determined by real time PCR. DNA containing the T7 promoter upstream of the luciferase gene was transcribed into RNA by the TNT T7 Quick for PCR DNA kit (Promega) at 30°C for 15-120 min. The reaction was stopped by immediately adding lysis buffer from the QIAMP Blood DNA mini kit (Qiagen) and incubating at 56° C for 10 min. This lysis buffer contains Proteinase K which should completely destroys RNA polymerase and thereby terminate the transcription of the DNA. RNA was purified using the same Blood DNA purification kit, which is known to copurify RNA efficiently. The purified RNA was further treated with TURBO DNA-*free*™ (Ambion) to completely remove residual DNA. The amount of RNA transcripts were then determined by quantitative RT-PCR using oligonucleotides 5'-GGAAGTCGGGGAAGCG-3' and 5'-TCTCACACACAGTTCGCCTC-3'. RT-PCR was performed using the Quantitect SybrGreen RT-PCR Kit (Qiagen) in a total volume of 20 µl with 5 µl (1:1000 diluted) purified RNA and each primer in a final concentration of 0.5 µM. All quantitative PCRs were performed on a Rotor-Gene 3000 (Corbett Research). The temperature profile was: 50° C for 20 min for reverse transcription, 94° C for 15 min, 40 cycles of 94° C for 15 s, 60° C for 30 s, 72° C for 30 s and acquiring SybrGreen flourescence at 81° C were performed followed by standard melting curve analysis of products. Appropriate no RT and no template controls were used for each experiment. A standard curve was used to calculate copy numbers of the transcripts. After a lag phase of 30 minutes, RNA levels were at least 10-fold higher for the C62 T7 promoter compared to the wild type T7 promoter (Fig. 5B).

*Translational activity of C62 T7 promoter variant:* To determine the translational activity of the C62 T7 promoter sequence, DNA was transcribed to RNA using the Ampliscribe High Yeild T7 transcription kit (Epicentre) as recommended by the manufacturer. The purified RNA was further treated with TURBO DNA-*free*™ (Ambion) to completely remove residual DNA. The RNA transcripts were quantified photometrically. 2 ng of purified RNA transcripts were then added to the TNT T7 Quick for PCR DNA kit (Promega). Expression of luciferase was quantified as described above. No differences in luciferase activities between wild type and C62 T7 promoter were observed (Figure 5C) indicating that the C62 T7 promoter does not affect translation efficiencies.

### Example 6: Activity of C62 T7 promoter based derivatives

The luciferase activity of C62 T7 promoter based derivatives was determined after IVTT. The C62 T7 promoter based derivatives comprise each one different nucleotide in the region of the last 10 nucleic acids at the 3' hydroxyl end compared to C62 T7 promoter. Said sequence of the C62 T7 promoter (the last 10 nucleic acids at the 3' hydroxyl end) is the randomized region compared to the wild type consensus sequence of the T7 promoter. The derivatives were produced by site-directed mutagenesis of the C62 T7 luciferase construct. To this end, the QuikChange site-directed mutagenesis kit (Stratagene) was used according to the manufacturer's protocol. In order to determine the luciferase activity of the different derivatives the protocol as described above was used. Only two of the 14 C62 T7 promoter derivatives remain an activity that was as high as or higher than the wild type T7 promoter (Figure 6). Hence, the only nucleotides that allow for a substitution are the cytosine at position +2 and the guanine at position +3. Said nucleotides can be substituted by guanine or cytosine, respectively, and still remain an activity that was as high as or higher than the wild type T7 promoter. Derivatives having mutations in other nucleotides of the randomized T7 promoter region show reduced promoter activity to about 1% of the activity of the C62 T7 promoter variant.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. Other embodiments are within the following claims. In addition, where features or aspects of the invention are described in terms of Markush groups, those skilled in the art will recognize that the invention is also thereby described in terms of any individual member or subgroup of members of the Markush group.

One skilled in the art would readily appreciate that the present invention is well adapted to carry out the objects and obtain the ends and advantages mentioned, as well as those inherent therein. Further, it will be readily apparent to one skilled in the art that varying substitutions and modifications may be made to the invention disclosed herein without departing from the scope and spirit of the invention. The compositions, methods, procedures, treatments, molecules and specific compounds described herein are presently representative of preferred embodiments are exemplary and are not intended as limitations on the scope of the invention. Changes therein and other uses will occur to those skilled in the art which are encompassed within the spirit of the invention are defined by the scope of the claims. The listing or discussion of a previously published document in this specification should not necessarily be taken as an acknowledgement that the document is part of the state of the art or is common general knowledge.

The invention illustratively described herein may suitably be practiced in the absence of any element or elements, limitation or limitations, not specifically disclosed herein. Thus, for example, the terms "comprising", "including," containing", etc. shall be read expansively and without limitation. The word "comprise" or variations such as "comprises" or "comprising" will accordingly be understood to imply the inclusion of a stated integer or groups of integers but not the exclusion of any other integer or group of integers. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by exemplary embodiments and optional features, modification and variation of the inventions embodied therein herein disclosed may be resorted to by those skilled in the art, and that such modifications and variations are considered to be within the scope of this invention.

The content of all documents and patent documents cited herein is incorporated by reference in their entirety.

## Claims

1. An isolated nucleic acid molecule comprising at least one sequence selected from the group consisting of SEQ ID NO:1, SEQ ID NO:2 and complements thereof.

2. The isolated nucleic acid molecule according to claim 1, wherein S at position 6 of SEQ ID NO:1 and S at position 19 of SEQ ID NO:2 is C and S at position 7 of SEQ ID NO:1 and S at position 20 of SEQ ID NO:2 is G.

3. The isolated nucleic acid molecule according to any one of claims 1 or 2, wherein the isolated nucleic acid molecule comprises SEQ ID NO:2.

4. The isolated nucleic acid molecule according to any one of claims 1 to 3, wherein said isolated nucleic acid molecule further comprises at least one of a nucleic acid sequence encoding for an open reading frame of a gene of interest, a -35/-10 box, a Shine-Dalgarno element, a TATA box, a CAAT sequence, a 5'-capping element, enhancer and/or repressor element(s), signal and/or leader sequence(s), a protein binding sequence and a restriction enzyme site.

5. A vector comprising the isolated nucleic acid molecule according to any one of claims 1 to 4.

6. The vector of claim 5, wherein said vector is a mammalian expression vector, a bacterial plasmid, a bacteriophage vector, a yeast episomal vector, an artificial chromosomal vector, or a viral vector.

7. A host cell comprising the isolated nucleic acid molecule or the vector according to any one of claims 1 to 6.

8. The host cell according to claim 7, wherein said host cell is a eukaryotic cell.

9. The host cell according to claim 7, wherein said host cell is a prokaryotic cell.

10. A method of producing RNA or protein, the method comprising
contacting the isolated nucleic acid molecule of claim 1 or the vector of claim 5 with an *in vitro* transcription translation reaction (IVTT) or an *in vitro* transcription reaction, or
introducing the isolated nucleic acid molecule of claim 1 or the vector of claim 5 into a host cell.

11. The method according to claim 10, the method comprising contacting the isolated nucleic acid of claim 1 or the expression vector of claim 5 with an *in vitro* transcription translation reaction (IVTT) or an *in vitro* transcription reaction.

12. The method according to claims 10 or 11, wherein the *in vitro* transcription translation reaction is a rabbit reticulocyte lysate.

13. The method according to claim 10, wherein said host cell is a eukaryotic cell.

14. The method according to claim 10, wherein said host cell is a prokaryotic cell.

15. A kit comprising the isolated nucleic acid molecule of claim 1 and/or the vector of claim 5.
